# EUROPÄISCHE PATENTANMELDUNG

(11) **EP 0 549 531 A1**
(43) Veröffentlichungstag der Anmeldung: **30.06.1993**
(21) Anmeldenummer: 92810994.1
(22) Anmeldetag: 11.12.1992
(51) Int. Cl.: C07C 51/567

(54) **Verfahren zur Herstellung von Säureanhydriden**

(30) Priorität: 20.12.1991 CH 3813/91
(71) Anmelder: Säurefabrik Schweizerhall, CH-4133 Schweizerhalle (CH)
(72) Erfinder: Gallegra , Pasquale, Dr., CH-4132 Muttenz (CH); Degischer, Gerhard, Dr., CH-4414 Füllinsdorf (CH)
(74) Vertreter: Schluep, Hans-Peter

(57) **Zusammenfassung**

Die Erfindung betrifft ein neuartiges Verfahren zur Herstellung von symmetrischen Säureanhydriden der Formel 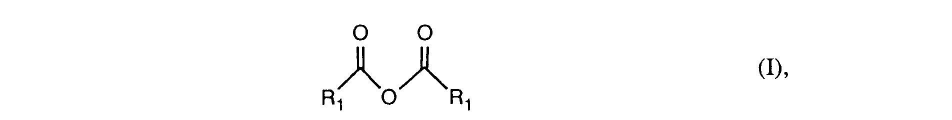
dadurch gekennzeichnet, dass man eine Verbindung der Formel 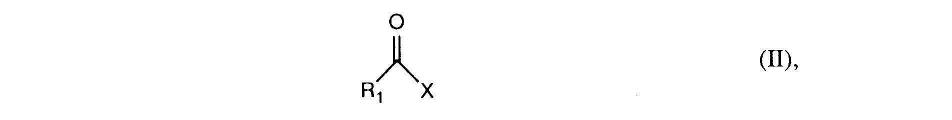
mit einem Säureanhydrid der Formel 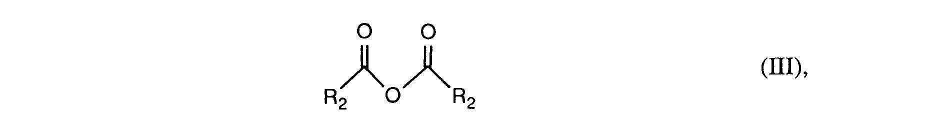
unter destillativer Entfernung der entstehenden Verbindungen der Formel 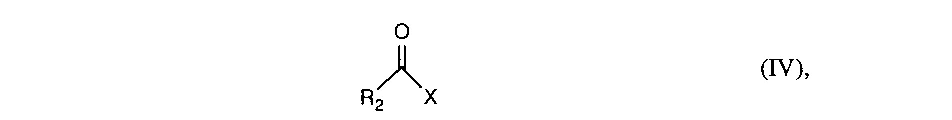
umsetzt. Die Reste R₁ und R₂ haben die in der Beschreibung angegebenen Bedeutungen, X bedeutet Chlor oder Brom.

## Beschreibung

Die vorliegende Erfindung betrifft ein neuartiges Verfahren zur Herstellung bestimmter Carbonsäurederivate, nämlich symmetrischer Anhydride.

Methoden zur Herstellung von symmetrischen Carbonsäureanhydriden sind beschrieben, beispielsweise in Houben-Weyl, "Methoden der Organischen Chemie", Erweiterungs- und Folgeband zur 4. Auflage, Band E5 "Carbonsäuren und Carbonsäure-Derivate", Stuttgart/New York 1985, S. 633 bis 652.

Es besteht ein Bedürfnis nach wirtschaftlichen Methoden zur Herstellung symmetrischer Carbonsäureanhydride, wobei insbesondere eine einfache Rektionsführung und Aufarbeitung wichtige Ziele sind. Sehr erstrebenswert sind solche Verfahren, die eine verbesserte Ausbeute an Endprodukt sowie dessen gute und einfache Abtrennung ermöglichen.

Die Erfindung betrifft ein neuartiges Verfahren zur Herstellung symmetrischer Säureanhydride der allgemeinen Formel 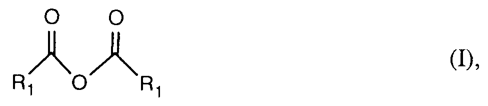
worin R₁ einen aliphatischen, cycloaliphatischen, aromatischen, cycloaliphatischaliphatischen oder araliphatischen Rest bedeutet, mit der Massgabe, dass im Rest R₁ vorhandene funktionelle Gruppen, wenn erforderlich, in geschützter Form vorliegen,
dadurch gekennzeichnet, dass man
eine Verbindung der Formel 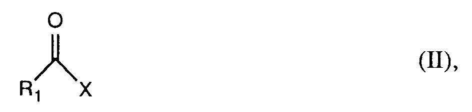
worin R₁ die oben genannten Bedeutungen hat und X Chlor oder Brom bedeutet, mit einem Säureanhydrid der Formel 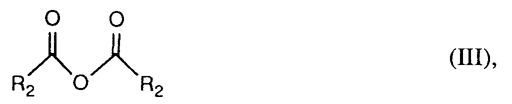
worin R₂ einen aliphatischen Kohlenwasserstoffrest oder Cycloalkyl bedeutet, unter destillativer Entfernung der entstehenden Verbindung der Formel 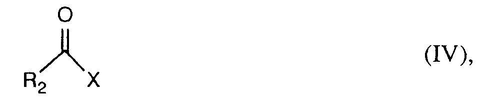
worin R₂ und X die oben angegebenen Bedeutungen haben, umsetzt, und vorhandene Schutzgruppen abspaltet.

Das erfindungsgemässe Verfahren erlaubt es, aus Säurechloriden die entsprechenden symmetrischen Säureanhydride durch Umsetzung mit Säureanhydriden anderer Struktur mit sehr hoher Ausbeute und Reinheit herzustellen. Dabei entstehen als Nebenprodukte die synthetisch wertvollen Säurechloride/-bromide der Formel IV.

Das erfindungsgemässe vereinfachte Verfahren zur Herstellung von symmetrischen Carbonsäureanhydriden besitzt insgesamt eine ganze Reihe von Vorteilen: Es kann in einer Eintopfreaktion ausgeführt werden, die verwendeten Ausgangsstoffe, insbesondere die Säurechloride und -bromide der Formel II, sind leicht zugänglich, und es liefert direkt die Carbonsäureanhydride der Formel I in sehr guter, praktisch quantitativer Ausbeute und hoher Reinheit, ohne dass störende oder aus Umweltschutzgründen bedenkliche Nebenprodukte gebildet werden, im Gegenteil: Es entstehen als Nebenprodukte Säurechloride oder -bromide der Formel IV, die ihrerseits sehr begehrte Reagentien für die organische Synthese darstellen und direkt rein oder in leicht zu reinigender Form gewonnen werden. Ein zusätzlicher bedeutender Vorteil des erfindungsgemässen Verfahrens besteht darin, dass die Aufarbeitung in verblüffend einfacher Weise erfolgt: Destillation reicht aus. Das Nebenprodukt der Formel IV, das während der Reaktion entsteht, wird in jedem Falle destillativ entfernt, vorzugsweise kontinuierlich während der Reaktion.

In der Beschreibung der vorliegenden Erfindung bedeutet der bei der Definition von Gruppen und Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die in der Beschreibung der vorliegenden Erfindung verwendeten allgemeinen Ausdrücke und Bezeichnungen haben vorzugsweise die folgenden Bedeutungen:

Ein aliphatischer Rest R₁ enthält beispielsweise bis zu 20 Kohlenstoffatome und ist gesättigt oder teilweise ungesättigt.

Ein bevorzugter aliphatischer Rest R₁ ist unsubstituiert und enthält dann mindestens 2 bis zu 20 Kohlenstoffatome, und ist vor allem ein gesättigter aliphatischer Kohlenwasserstoffrest, in erster Linie Alkyl, welches geradkettig oder ein- oder mehrfach verzweigt ist, ausser Methyl, insbesondere C₂-C₇-Alkyl, wie Ethyl, n-Propyl, Isopropyl, Butyl, Isobutyl, sec-Butyl, tert-Butyl, Pentyl, Isopentyl, sec-Pentyl, Neopentyl, Hexyl oder Heptyl, wobei tert-Butyl sehr bevorzugt ist, ferner Octyl, Nonyl oder Decyl, oder ein ungesättigter Kohlenwasserstoffrest, in erster Linie Alkenyl, insbesondere Niederalkenyl, z.B. geradkettiges oder verzweigtes C₂-C₇-Alkenyl, wie Vinyl, Propenyl, z.B. Allyl, Isopropenyl, Crotyl, 1-Butenyl oder 2-Methyl-prop2-enyl, oder Alkinyl, z.B. C₃-C₇-Alkinyl, wie Propargyl. Ganz besonders bevorzugt ist tert-Butyl R₁.

Ein bevorzugter gesättigter aliphatischer Rest R₁ ist auch substituiertes geradkettiges oder ein- oder mehrfach verzweigtes C₁-C₂₀-Alkyl, insbesondere subtituiertes Niederalkyl, wie Methyl, Ethyl, n-Propyl oder Isopropyl, Butyl, Isobutyl, sec-Butyl, tert-Butyl, Pentyl, Isopentyl, sec-Pentyl, Neopentyl, Hexyl oder Heptyl, oder ferner substituiertes Octyl, Nonyl oder Decyl. Bevorzugte Substituenten sind einer oder mehrere, insbesondere bis zu drei, z.B. einer, der Reste Niederalkoxy, wie Methoxy, Niederalkanoyloxy, wie Acetoxy, Phenyloxy, Niederalkylmercapto, wie Methylmercapto, Niederalkoxycarbonyl, wie Methoxycarbonyl oder tert-Butoxycarbonyl, Halogen, wie Fluor, Chlor, Brom oder Iod, Oxo und/oder Nitro. Bevorzugte Substituenten sind auch solche funktionelle Gruppen, die während der Reaktion in geschützter Form vorliegen müssen, wie Hydroxy, Mercapto, Amino, Niederalkylamino, wie Methylamino, oder Carboxy. Besonders bevorzugte Substituenten sind Niederalkoxy, wie Methoxy, Phenyloxy, Niederalkylmercapto, wie Methylmercapto, Diniederalkylamino, wie Dimethylamino, Halogen, wie Fluor, Chlor, Brom oder Iod, Oxo und/oder Nitro, ferner weitere Substituenten, für die im erfindungsgemässen Verfahren keine Schutzgruppen erforderlich sind. Ganz besonders bevorzugt ist durch Halogen substituiertes Niederalkyl R₁, wie Chlor- oder Brommethyl, insbesondere Chlormethyl.

Ein bevorzugter cycloaliphatischer Rest R₁ enthält 3 bis 20 Kohlenstoffatome und ist mono-, bi- oder tricyclisch, insbesondere C₃-C₈-Cycloalkyl, wie Cyclopropyl, Cyclobutyl, oder, vorzugsweise, C₅-C₇-Cycloalkyl, wie Cyclopentyl, Cyclohexyl oder Cycloheptyl, wobei die genannten Reste durch die bei der Definition eines aliphatischen Restes R₁ genannten Substituenten oder ferner durch Niederalkyl, wie Methyl, substituiert oder, vorzugsweise, unsubstituiert sind. Stark bevorzugt ist Cyclohexyl.

Ein bevorzugter aromatischer Rest R₁ enthält bis zu 20, vorzugsweise bis zu 14, Kohlenstoffatome und ist insbesondere ausgewählt aus Phenyl, Naphthyl, wie 1- oder 2-Naphthyl, Indenyl, wie 2- oder 4-lndenyl, Indanyl, wie 2-lndanyl, Anthryl, wie 1- oder 2-Anthryl, Phenanthryl, wie 9-Phenanthryl, Acenaphthenyl, wie 1-Acenaphthenyl, oder Fluorenyl, wie 9-Fluorenyl, vorzugsweise Phenyl oder Naphthyl, wobei die genannten Reste wie bei der Definition eines aliphatischen Restes R₁ oder ferner durch Alkyl, insbesondere durch Niederalkyl, Niederalkoxy, Halogen, Trifluormethyl und/oder Nitro, substituiert oder, vorzugsweise, unsubstituiert sind. Ganz besonders bevorzugt ist Phenyl.

Ein bevorzugter cycloaliphatisch-aliphatischer Rest R₁ ist einer der bei der Definition aliphatischer Reste R₁ genannten, substituierten oder, vorzugsweise, unsubstituierten Reste, insbesondere Niederalkyl, wie Methyl, Ethyl, Propyl oder Butyl, der mit einem der bei der Definition cycloaliphatischer Reste R₁ genannten, substituierten oder, vorzugsweise, unsubstituierten Reste, insbesondere C₃-C₈-Cycloalkyl, wie Cyclopentyl, Cyclohexyl oder Cycloheptyl, verknüpft ist, wobei der cycloaliphatische Rest vorzugsweise endständig an den aliphatischen Rest gebunden ist. Stark bevorzugt ist Cyclohexylmethyl.

Ein bevorzugter araliphatischer Rest R₁ ist einer der bei der Definition aliphatischer Reste R₁ genannten, substituierten oder, vorzugsweise, unsubstituierten Reste, insbesondere Niederalkyl, wie Methyl, Ethyl, Propyl oder Butyl, der mit einem oder mehreren der bei der Definition aromatischer Reste R₁ genannten, substituierten oder, vorzugsweise, unsubstituierten Reste, insbesondere Phenyl oder Napthyl, verknüpft ist, wobei als Substituenten für den aromatischen Rest Niederalkyl, Niederalkoxy, Halogen, wie Fluor, Chlor oder Brom, Trifluormethyl und/oder Nitro besonders bevorzugt sind und der aromatische Rest einfach, zweifach oder dreifach vorliegen kann, wie in Mono, Di- oder Triphenylniederalkyl, oder vorzugsweise einfach und endständig an den aliphatischen Rest gebunden vorliegt. Stark bevorzugt ist Phenylmethyl.

Funktionelle Gruppen, die in geschützter Form vorliegen, sind solche, die nicht an der Reaktion teilnehmen sollen, wie Hydroxy-, Mercapto, Amino-, Mononiederalkylamino- oder Carboxygruppen. Diese funktionellen Gruppen können durch geeignete Schutzgruppen (conventional protecting groups) geschützt sein, wie sie üblicherweise bei der Synthese von Peptid-Verbindungen, aber auch von Cephalosporinen und Penicillinen, Nucleinsäuren und Zuckern, verwendet werden. Diese Schutzgruppen können bereits in den Vorstufen vorhanden sein und sollen die betreffenden funktionellen Gruppen gegen unerwünschte Nebenreaktionen wie Acylierungen, ferner Veretherungen, Veresterungen, Oxidationen, etc., schützen. Charakteristisch für Schutzgruppen ist, dass sie leicht, d.h. ohne unerwünschte Nebenreaktionen, abspaltbar sind. Als Schutzgruppen werden nur diejenigen Gruppen bezeichnet, die nicht unter die Definition der Endprodukte der Formel I fallen.

Die Abspaltung der Schutzgruppen, die nicht Bestandteil des gewünschten Endproduktes der Formel I sind, erfolgt in an sich bekannter Weise, z.B. mittels Solvolyse, Acidolyse oder Reduktion, oder auch enzymatisch, gegebenenfalls stufenweise oder unter gleichzeitiger Abspaltung aller abzuspaltenden Schutzgruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, in "Methoden der organischen Chemie", Houben-Weyl, 4. Auflage, Bd. 15/I, Georg Thieme Verlag, Stuttgart 1974, in H.-D. Jakubke und H. Jescheit, "Aminosäure, Peptide, Proteine", Verlag Chemie, Weinheim, Deerfield Beech und Basel 1982, und in Jochen Lehmann, "Chemie der Kohlenhydrate: Monosaccharide und Derivate", Georg Thieme Verlag, Stuttgart 1974, beschrieben.

Eine Hydroxygruppe wird beispielsweise durch eine durch Halogen, z.B. Chlor, substituierte Niederalkanoylgruppe, z.B. 2,2-Dichloracetyl, durch 2-Chlorethoxycarbonyl, 2,2,2-Trichlorethoxycarbonyl, 4-Nitrobenzyloxycarbonyl oder Diphenylmethoxycarbonyl, durch Triniederalkylsilyl, z.B. Trimethylsilyl oder bevorzugt tert-Butyl-dimethylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5 - 7 Ringatomen, wie 2-Tetrahydrofuryl oder 2-Tetrahydropyranyl, ein entsprechendes Thiaanaloges, oder durch 1-Phenylniederalkyl, wie Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt.

Eine Mercaptogruppe wird beispielsweise durch ein im Phenylrest, z.B. durch Methoxy oder Nitro, substituiertes Benzyl, wie 4-Methoxybenzyl, ein gegebenenfalls im Phenylrest, z.B. durch Methoxy, substituiertes Diphenylmethyl, wie Di-(4-methoxyphenyl)-methyl, Triphenylmethyl, durch Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo geschützt.

Eine Amino- oder Mononiederalkylaminogruppe wird beispielsweise geschützt durch Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Iod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl, Di-(4-methoxyphenyl)-methoxycarbonyl oder 9-Fluorenylmethoxycarbonyl, Aroylmethoxycarbonyl, z.B. Phenacyloxycarbonyl, 2-Halogen-niederalkoxycarbonyl, z.B. 2-Chlorethoxycarbonyl, 2,2,2-Trichlorethoxycarbonyl, 2-Bromethoxycarbonyl oder 2-lodethoxycarbonyl, 2-Triniederalkylsilylniederalkoxycarbonyl, z.B. 2-Trimethlysilylethoxycarbonyl, oder 2-Triarylsilylniederalkoxycarbonyl, z.B. 2-Triphenylsilylethoxycarbonyl.

Eine Carboxygruppe wird beispielsweise geschützt, indem sie durch eine Niederalkylgruppe verestert ist, die in 1-Stellung der Niederalkylgruppe verzweigt ist, beispielsweise in tert-Niederalkoxycarbonyl, z.B. tert-Butoxycarbonyl, oder in Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy, Halogen, z.B. Chlor, und- oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Ein bevorzugter aliphatischer Kohlenwasserstoffrest R₂ ist Methyl oder ein ungesättigter oder, insbesondere, ein gesättigter Kohlenwasserstoffrest, wie für unsubstituierte aliphatische Reste R₁ oben definiert. Besonders bevorzugt ist Niederalkyl R₂, wie Methyl, Ethyl, n-Propyl, Isopropyl, n-Butyl, sec-Butyl oder tert-Butyl. Ganz besonders bevorzugt ist R₂ Methyl.

Bevorzugtes Cycloalkyl R₂ ist C₃-C₆-Cycloalkyl, wie Cyclopropyl, Cyclobutyl, oder, insbesondere, Cyclopentyl oder Cyclohexyl.

Wie sich aus der Bedingung, dass die Verbindungen der Formel IV destillativ entfernt werden, zwangsläufig ergibt, ist bei der Definition der Reste R₁ und R₂ stets folgendes zu berücksichtigen: Die Reste R₁ und R₂ müssen so gewählt sein, dass der Siedepunkt der Verbindung der Formel I, vorzugsweise auch der Verbindungen der Formeln II und III und eventueller Zwischenstufen im Reaktionsgemisch, ausreichend viel höher ist als der Siedepunkt der Verbindungen der Formel IV, um ein Abdestillieren der Verbindungen der Formel IV während der Reaktion zu ermöglichen. Vorzugsweise haben die Verbindungen der Formel IV unter den jeweiligen Reaktionsbedingungen einen um wenigstens 10 °C niedrigeren Siedepunkt als die der Formel II.

Die Verbindungen der Formel I sind wertvolle Zwischenprodukte in der organischen Synthese und werden häufig als Ersatzchemikalien für die Säurechloride oder -bromide der Formel II eingesetzt, da sie eine etwas geringere, jedoch noch vergleichbare Reaktivität und andererseits eine höhere Selektivität als die Verbindungen der Formel II aufweisen. So verursachen die Säureanhydride der Formel I beispielsweise im grosstechnischen Einsatz nicht die bei der Verwendung von Säurechloriden auftretende Korrosion. Auch die Verwendung der Carbonsäureanhydride als Zwischenprodukte für Wirkstoffe des Pflanzenschutzes ist möglich; z.B. können die Säureanhydride der Formel I als Ausgangsstoffe für die Herstellung von Acylcyaniden (vgl. z.B. DE-OS 26 14 240, DE-OS 26 14 241, DE-OS 26 42 140, DE-OS 26 42 199) verwendet werden, die ihrerseits als Zwischenprodukte, beispielsweise zur Synthese von 1,2,4-Triazin-5-onen, Verbindungen mit hervorragenden herbiziden Eigenschaften, eingesetzt werden (vgl. DE-OS 27 33 180, DE-OS 30 03 541, DE-OS 30 08 921, DE-OS 30 02 203, DE-OS 30 09 043).

So lässt sich z.B. Benzoesäureanhydrid nach einem bekannten Verfahren in den herbiziden Wirkstoff 3-Methyl-4-amino-6-phenyl-1,2,4-triazin-5-on (geläufiger Name: Metamitron) umwandeln, indem man in einer ersten Stufe Benzoesäureanhydrid durch Umsetzung mit Alkalimetallcyaniden oder wasserfreier Blausäure in Benzoylcyanid überführt, dieses in einer zweiten Stufe in Gegenwart von konzentrierter Salzsäure mit Ethanol umsetzt und den dabei entstehenden Phenylglyoxalsäureethylester in einer dritten Stufe mit Acetylhydrazin zur Reaktion bringt, wobei sich 1 -Phenyl-glyoxylsäureethylester2-acetylhydrazon bildet, das in einer vierten Stufe mit Hydrazinhydrat in Gegenwart von Pyridin in das oben erwähnte Endprodukt überführt wird (vgl. z.B. DE-OS 22 24 161, DE-OS 26 14 240, DE-OS 26 14 241).

Nach ebenfalls bekannten Verfahren kann Pivalinsäureanhydrid in den herbiziden Wirkstoff 3-Methylthio-4-amino-6-tert-butyl- 1,2,4-triazin-5-on (geläufiger Name: Metribuzin) überführt werden (vgl. z.B. DE-OS 26 14 240 und DE-OS 26 14 241 in Verbindung mit DE-OS 30 09 043).

Die Erfindung betrifft in erster Linie die Herstellung von Verbindungen der Formel I, worin R₁ C₂-C₇-Alkyl, durch Niederalkoxy oder Halogen substituiertes Niederalkyl, C₂-C₇-Alkenyl, C₃-C₇-Alkinyl, C₃-C₈-Cycloalkyl, unsubstituiertes oder durch Niederalkyl, Niederalkoxy, Halogen, Trifluormethyl und/oder Nitro substituiertes Phenyl oder Naphthyl, oder im Phenylteil unsubstituiertes oder durch Niederalkyl, Niederalkoxy, Halogen, Trifluormethyl und/oder Nitro substituiertes Mono-, Di- oder Triphenylniederalkyl bedeutet.

Die Erfindung betrifft insbesondere die Herstellung von Verbindungen der Formel I, worin R₁ C₂-C₇-Alkyl oder durch Halogen substituiertes Niederalkyl, wie tert-Butyl oder Chlormethyl, C₅-C₇-Cycloalkyl, Phenyl oder Naphthyl bedeutet.

Die Erfindung betrifft vorzugsweise die Herstellung von Verbindungen der Formel I, worin R₁ C₂-C₇-Alkyl, durch Fluor, Chlor oder Brom bis zu dreifach substituiertes Niederalkyl oder Phenyl bedeutet.

Die Erfindung betrifft in besonders bevorzugter Weise die Herstellung von Verbindungen der Formel I, worin R₁ tert-Butyl, Chlormethyl oder Phenyl bedeutet.

R₁ in den Ausgangsverbindungen der Formel II ist jeweils entsprechend definiert.

In den Säureanhydriden der Formel III ist R₂ in erster Linie Niederalkyl oder C₃-C₆-Cycloalkyl.

Insbesondere ist R₂ Niederalkyl.

Besonders bevorzugt ist als Verbindung der Formel II Essigsäureanhydrid (R₂ = Methyl).

R₂ in den Verbindungen der Formel IV ist jeweils entsprechend definiert.

Die Umsetzung einer Verbindung der Formel II mit einem Säureanhydrid der Formel III erfolgt in erster Linie unter Verwendung der gegenüber dem Säureanhydrid der Formel III mindestens zweifachen molaren Menge einer Verbindung der Formel II, in An- oder Abwesenheit von Lösungs- oder Verdünnungsmitteln, vorteilhaft bei erhöhter Temperatur, und unter Bedingungen, die eine destillative Entfernung der entstehenden Verbindung der Formel IV ermöglichen, bei geeigneten Drucken, in An- oder Abwesenheit von Schutzgasen, sowie gegebenenfalls unter anschliessender weiterer Aufarbeitung.

Eine gegenüber dem Säureanhydrid der Formel III mindestens zweifache molare Menge einer Verbindung der Formel II bedeutet in erster Linie, dass das molare Verhältnis der Verbindungen der Formel II zu den Verbindungen der Formel III zwischen 2 und 10, insbesondere zwischen 2 und 5, z.B. zwischen 2 und 3, wobei die Grenzwerte jeweils eingeschlossen sind, liegt.

Als Lösungs- oder Verdünnungsmittel kommen in erster Linie aprotische, inerte Lösungsmittel zum Einsatz, die ausreichend viel höher sieden als die jeweilige Verbindung der Formel IV, um zu gewährleisten, dass diese Verbindung während der Reaktion ständig abdestilliert werden kann, vorzugsweise um wenigstens 10 °C höher. Denkbar sind aber auch solche Lösungsmittel, die niedriger sieden als die Verbindungen der Formel IV, so dass das Lösungsmittel beispielsweise auch als Schlepper für die Abdestillation von Verbindungen der Formel IV verwendet werden kann, wobei, falls erforderlich, die Reaktion unterbrochen werden kann, um das Lösungsmittel nachzufüllen. Besonders bevorzugt sind solche Lösungsmittel, die höher sieden als die abzudestillierende Verbindung der Formel IV, in erster Linie um wenigstens 10 °C höher, aber zugleich eine niedrigere Siedetemperatur haben als die Produkte der Formel I, so dass das Lösungsmittel nach der Reaktion aus dem Reaktionsgefäss destillativ entfernt werden kann, in erster Linie um wenigstens 10 °C niedriger. Zu den Lösungsmitteln, aus denen ausgewählt werden kann, zählen vor allem Kohlenwasserstoffe, wie Alkane, z.B. Hexan, Pentan, Heptan oder Octan, Halogenalkane, wie Di-, Tri- oder Tetrachlor-C₁-C₄-alkane, z.B. Methylenchlorid oder Trichlorethan, Aromaten, wie Benzol oder Toluol, Halogenaromaten, wie Chlorbenzol, oder Ether, wie Dioxan. Vorteilhaft kann die erfindungsgemässe Umsetzung jedoch ohne Lösungsmittel vorgenommen werden, beispielsweise in einer Schmelze oder insbesondere dann, wenn wenigstens das verwendete Säureanhydrid der Formel III bei der jeweiligen Reaktionstemperatur flüssig ist, so dass es gleichzeitig als Reagens und als Solvens vorliegt. Eine ganz besonders geeignete Verbindung der Formel III ist Essigsäureanhydrid.

Die Temperatur der Reaktion liegt zwischen 10 °C und Siedetemperatur des Reaktionsgemisches, wobei die höchstzulässige Reaktionstemperatur so zu wählen ist, dass keine unerwünschten Nebenreaktionen eintreten können. Vorzugsweise liegt die Temperatur zwischen Raumtemperatur und einer maximalen Temperatur von 200 °C, z.B. zwischen 60 und 180 °C, hauptsächlich bei 10 bis 120 °C.

Das Nebenprodukt der Formel IV, das während der Reaktion entsteht, wird in jedem Falle destillativ entfernt, vorzugsweise kontinuierlich während der Reaktion.

Zur Erfüllung der Bedingung, dass eine destillative Entfernung der Verbindung der Formel IV ermöglicht wird, dienen bei Anwesenheit weiterer Lösungs- und Verdünnungsmittel die oben bei deren Definition genannten Beschränkungen. Bei der bevorzugten Reaktionsführung ohne Lösungsmittel ist, wie oben bei der Definition von R₁ und R₂ erwähnt, Voraussetzung, dass diese Reste so gewählt sein müssen, dass der Siedepunkt der Verbindung der Formel I, vorzugsweise auch derjenige der Verbindungen der Formeln II und III, stets ausreichend viel höher liegt als der Siedepunkt der Verbindungen der Formel IV, um ein Abdestillieren der Verbindungen der Formel IV während der Reaktion zu ermöglichen. Vorzugsweise hat eine Verbindung der Formel IV unter den jeweiligen Reaktionsbedingungen einen um wenigstens 10 °C niedrigeren Siedepunkt als die Verbindung der Formel II. Eine ganz besonders geeignete Verbindung der Formel III ist daher Essigsäureanhydrid.

Geeignete Drucke zur Reaktionsführung sind vorzugsweise Atmosphärendruck oder, insbesondere bei Reaktionsgemischen, deren Siedepunkt höher ist als der nach der Definition der höchstzulässigen Temperatur oben genannte Wert, verminderte Drucke, vorzugsweise von 1 mbar bis Atmosphärendruck, vorteilhaft 10 bis 900 mbar oder Atmosphärendruck, insbesondere 100 bis 600 mbar oder Atmosphärendruck, z.B. etwa 400 mbar oder Atmosphärendruck.

Die Reaktion kann mit oder, vorzugsweise, ohne Gegenwart von Schutzgas, wie Stickstoff oder Argon, insbesondere Stickstoff, erfolgen.

Die anschliessende weitere Aufarbeitung erfolgt vorzugsweise durch destillative Entfernung überschüssiger Lösungs- und Verdünnungsmittel und, falls ein Überschuss der Verbindung der Formel II vorliegt, durch dessen, vorzugsweise destillative, Entfernung. Die destillative Entfernung erfolgt bei Atmosphärendruck oder bei vermindertem Druck, je nach dem Siedepunkt der Ausgangsverbindung der Formel II, vorzugsweise bei vermindertem Druck, vorteilhaft bei 1 bis 1000 mbar, insbesondere bei 5 bis 500 mbar, wie zwischen 2 und 200 mbar, z.B. bei etwa 5 bis 10 oder bei etwa 150 mbar. Ferner können geschützte funktionelle Gruppen, wie oben beschrieben, freigesetzt werden.

Das Ende der Reaktion kann durch übliche analytische Methoden, beispielsweise gaschromatographisch, ermittelt werden, oder einfach durch die Messung der durch derartige Analytik bestimmten Siedetemperatur des Reaktionsgemisches, bei der das gesamte Nebenprodukt der Formel IV abdestilliert ist.

Die Erfindung betrifft ganz besonders bevorzugt das Verfahren, worin Chloracetylchlorid der Formel II und Essigsäureanhydrid der Formel III im Molverhältnis von 2 : 1 bis 5 : 1 bei 100 bis 120 °C bei gleichzeitigem Abdestillieren des entstehenden Acetylchlorids der Formel IV umgesetzt werden unter Bildung von Chloressigsäureanhydrid der Formel I, welcher nach Abdestillieren des überschüssigen Chloracetylchlorides bei vermindertem Druck von 2 bis 200 mbar in reiner Form erhalten wird.

Die Erfindung betrifft auch ganz besonders bevorzugt das Verfahren, worin Benzoylchlorid der Formel II und Essigsäureanhydrid der Formel III im Molverhältnis von 2: 1 bis 5 : 1 bei 100 bis 120 °C bei gleichzeitigem Abdestillieren des entstehenden Acetylchlorids der Formel IV bei 100 bis 600 mbar umgesetzt werden unter Bildung von Benzoesäureanhydrid der Formel I, welcher nach Abdestillieren des überschüssigen Benzoylchlorides der Formel IV bei vermindertem Druck von 2 bis 200 mbar in reiner Form erhalten wird.

Die Erfindung betrifft schliesslich auch ganz besonders bevorzugt das Verfahren, worin Pivaloylchorid der Formel II und Essigsäureanhydrid der Formel III im Molverhältnis von 2 : 1 bis 5 : 1 bei 100 bis 120 °C bei gleichzeitigem Abdestillieren des entstehenden Acetylchlorids der Formel IV umgesetzt werden unter Bildung von Pivaloylanhydrid der Formel I, welcher nach Abdestillieren des überschüssigen Pivaloylchlorides bei vermindertem Druck von 2 bis 200 mbar in reiner Form erhalten wird.

Die Erfindung betrifft in allererster Linie die in den Beispielen beschriebene Herstellung der dort genannten Verbindungen der Formel I, namentlich von Chloressigsäureanhydrid (R₁ = Chlormethyl), Benzoesäureanhydrid (R₁ = Phenyl) und Pivalinsäureanhydrid (R₁ = tert-Butyl).

### Ausgangsverbindungen

In den Verbindungen der Formel II und deren Vorstufen können im Rest R₁ vorhandene funktionelle Gruppen, falls erforderlich, in geschützter Form vorliegen. Die Einführung und die Abspaltung von Schutzgruppen erfolgt, wie bei der Definition der Schutzgruppen beschrieben.

Die Ausgangsverbindungen der Formel II sind bekannt, werden nach den zur Herstellung von Carbonsäurechloriden und -bromiden üblichen Verfahren hergestellt, oder sind kommerziell erhältlich.

Beispielsweise können die entsprechenden freien Carbonsäuren durch Umsetzung mit anorganischen Säurechloriden, wie Phosphortrichlorid, Phosphorpentachlorid und Thionylchlorid, in die entsprechenden Carbonsäurechloride überführt werden. Zur Überführung empfindlicher Carbonsäuren in ihre Halogenide eignet sich auch die Umsetzung der freien Carbonsäuren mit Oxalyldichlorid. Die Säurebromide der Formel II können beispielsweise mit Hilfe analoger anorganischer Säurebromide oder aus den Säurechloriden durch Einwirkung von trockenem Bromwasserstoff erhalten werden.

Die Carbonsäureanhydride der Formel III sind ebenfalls bekannt, nach an sich bekannten Verfahren herstellbar oder kommerziell erhältlich.

Beispielsweise können sie nach einer der Methoden hergestellt werden, wie in Houben-Weyl, "Methoden der Organischen Chemie", Erweiterungs- und Folgeband zur 4. Auflage, Band E5 "Carbonsäuren und Carbonsäure-Derivate", Stuttgart/New York 1985, S. 633 bis 652, beschrieben sind.

Die nachfolgenden Beispiele dienen der Illustration der Erfindung, ohne deren Umfang einzuschränken; Temperaturen werden in Grad Celsius (°C), Drucke in mbar angegeben.

### Beispiel 1: Chloressigsäureanhydrid

613 g Essigsäureanhydrid (6 mol) werden mit 2000 g Chloracetylchlorid (17,7 mol) vermischt und auf 100 - 120 °C erwärmt. Das entstehende Acetylchlorid wird abdestilliert. Am Ende der Reaktion wird das überschüssige Chloracetylchlorid unter vermindertem Druck (etwa 150 mbar) destillativ entfernt. Der erhaltene Rückstand ist reines Chloressigsäureanhydrid. Ausbeute: 1016 g (= 99 % der Theorie) Chloressigsäureanhydrid.

### Beispiel 2: Benzoesäureanhydrid

300 g Benzoylchlorid (2,13 mol) werden mit 91 g Essigsäureanhydrid (0,89 mol) vermischt und auf 100 bis 120 °C erwärmt. Das entstehende Acetylchlorid wird unter vermindertem Druck (400 mbar) destillativ entfernt, überschüssiges Benzoylchlorid nach vollständigem Ablauf der Reaktion bei etwa 7 mbar. Der erhaltene Rückstand ist ist reines Benzoesäureanhydrid. Ausbeute: 200 g (= 99 % der Theorie) Benzoesäureanhydrid.

### Beispiel 3: Pivalinsäureanhydrid

400 g Essigsäureanhydrid (3,92 mol) werden mit 1200 g Pivaloylchlorid (9,95 mol) vermischt und auf 100-120 °C erwärmt. Das entstehende Acetylchlorid wird abdestilliert. Am Ende der Reaktion wird das überschüssige Pivaloylchlorid unter vermindertem Druck von etwa 150 mbar destillativ entfernt. Der erhaltene Rückstand ist reines Pivalinsäureanhydrid. Ausbeute: 722 g (= 99 % der Theorie) Pivalinsäureanhydrid.

## Patentansprüche

1. Verfahren zur Herstellung von symmetrischen Säureanhydriden der Formel 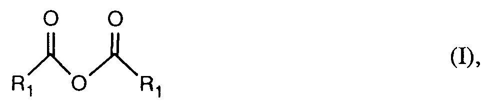 worin R₁ einen aliphatischen, cycloaliphatischen, aromatischen, cycloaliphatisch-aliphatischen oder araliphatischen Rest bedeutet, mit der Massgabe, dass im Rest R₁ vorhandene funktionelle Gruppen, wenn erforderlich, in geschützter Form vorliegen,
dadurch gekennzeichnet, dass man eine Verbindung der Formel 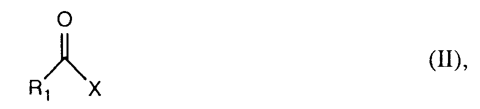 worin R₁ die oben genannten Bedeutungen hat und X Chlor oder Brom bedeutet, mit einem Säureanhydrid der Formel 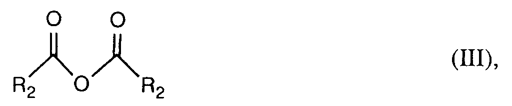 worin R₂ einen aliphatischen Kohlenwasserstoffrest oder Cycloalkyl bedeutet, unter destillativer Entfernung der entstehenden Verbindung der Formel 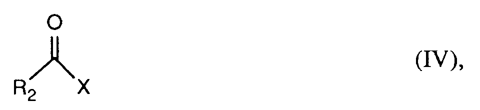 worin R₂ und X die oben angegebenen Bedeutungen haben, umsetzt, und vorhandene Schutzgruppen abspaltet.

2. Verfahren gemäss Anspruch 1, in dem man Verbindungen der Formeln II und III einsetzt, worin R₁ C₂-C₇-Alkyl, durch Niederalkoxy oder Halogen substituiertes Niederalkyl, C₂-C₇-Alkenyl, C₃-C₇-Alkinyl, C₃-C₈-Cycloalkyl, unsubstituiertes oder durch Niederalkyl, Niederalkoxy, Halogen, Trifluormethyl und/oder Nitro substituiertes Phenyl oder Naphthyl, oder im Phenylteil unsubstituiertes oder durch Niederalkyl, Niederalkoxy, Halogen, Trifluormethyl und/oder Nitro substituiertes Mono-, Di- oder Triphenylniederalkyl bedeutet und R₂ Niederalkyl oder C₃-C₆-Cycloalkyl bedeutet.

3. Verfahren gemäss Anspruch 1, in dem man Verbindungen der Formeln II und III einsetzt, worin R₁ C₂-C₇-Alkyl, durch Halogen substituiertes Niederalkyl, C₅-C₇-Cycloalkyl, Phenyl oder Naphthyl bedeutet und R₂ Niederalkyl bedeutet.

4. Verfahren gemäss einem der Ansprüche 1 bis 3, worin die Reaktionstemperatur zwischen Raumtemperatur und dem Siedepunkt des Reaktionsgemisches, jedoch nicht über 200 °C liegt.

5. Verfahren gemäss einem der Ansprüche 1 bis 4, worin das molare Verhältnis der Verbindungen der Formel II zu den Anhydriden der Formel III zwischen 2 und 10 liegt.

6. Verfahren gemäss einem der Ansprüche 1 bis 5, worin die Verbindung der Formel IV unter Atmosphärendruck oder bei vermindertem Druck abdestilliert wird.

7. Verfahren gemäss einem der Ansprüche 1 bis 6, worin die Reste R₂ in Säureanhydriden der Formel III so gewählt sind, dass der Siedepunkt der Verbindung der Formel IV um mehr als 10 °C unter dem der Verbindung der Formel II liegt, jedoch 200 °C nicht übersteigt.

8. Verfahren gemäss einem der Ansprüche 1 bis 7, worin für den Fall, dass ein Überschuss der Verbindungen der Formel II über die Säureanhydride der Formel III vorliegt, nach der Reaktion eine destillative Trennung der als Gemisch vorliegenden Verbindungen der Formeln I und II erfolgt.

9. Verfahren gemäss einem der Ansprüche 1 und 4 bis 8, in dem man Verbindungen der Formeln II und III einsetzt, worin R₁ C₂-C₇-Alkyl, durch Fluor, Chlor oder Brom bis zu dreifach substituiertes Niederalkyl oder Phenyl bedeutet und R₂ Niederalkyl bedeutet, wobei in den Verbindungen der Formeln II und IV X Chlor bedeutet.

10. Verfahren gemäss einem der Ansprüche 1 und 4 bis 8, in dem man Verbindungen der Formeln II und III einsetzt, worin R₁ tert-Butyl, Chlormethyl oder Phenyl bedeutet und R₂ Methyl bedeutet, wobei in den Verbindungen der Formeln II und IV X Chlor bedeutet.

11. Verfahren gemäss einem der Ansprüche 1 und 4 bis 8, in dem man Verbindungen der Formeln II und III einsetzt, worin R₁ tert-Butyl bedeutet und R₂ Methyl bedeutet, wobei in den Verbindungen der Formeln II und IV X Chlor bedeutet.

12. Verfahren gemäss einem der Ansprüche 1 und 4 bis 8, in dem man Verbindungen der Formeln II und III einsetzt, worin R₁ Chlormethyl bedeutet und R₂ Methyl bedeutet, wobei in den Verbindungen der Formeln II und IV X Chlor bedeutet.

13. Verfahren gemäss einem der Ansprüche 1 und 4 bis 8, in dem man Verbindungen der Formeln II und III einsetzt, worin R₁ Phenyl bedeutet und R₂ Methyl bedeutet, wobei in den Verbindungen der Formeln II und IV X Chlor bedeutet.

14. Verfahren gemäss Anspruch 1, worin Chloracetylchlorid der Formel II und Essigsäureanhydrid der Formel III im Molverhältnis von 2 : 1 bis 5 : 1 bei 100 bis 120 °C bei gleichzeitigem Abdestillieren des entstehenden Acetylchlorids der Formel IV umgesetzt werden unter Bildung von Chloressigsäureanhydrid der Formel I, welcher nach Abdestillieren des überschüssigen Chloracetylchlorides bei vermindertem Druck von 2 bis 200 mbar in reiner Form erhalten wird.

15. Verfahren gemäss Anspruch 1, worin Benzoylchlorid der Formel II und Essigsäureanhydrid der Formel III im Molverhältnis von 2 : 1 bis 5 : 1 bei 100 bis 120 °C bei gleichzeitigem Abdestillieren des entstehenden Acetylchlorids der Formel IV bei 100 bis 600 mbar umgesetzt werden unter Bildung von Benzoesäureanhydrid der Formel I, welcher nach Abdestillieren des überschüssigen Benzoylchlorides der Formel IV bei vermindertem Druck von 2 bis 200 mbar in reiner Form erhalten wird.

16. Verfahren gemäss Anspruch 1, worin Pivaloylchorid der Formel II und Essigsäureanhydrid der Formel III im Molverhältnis von 2: 1 bis 5 : 1 bei 100 bis 120 °C bei gleichzeitigem Abdestillieren des entstehenden Acetylchlorids der Formel IV umgesetzt werden unter Bildung von Pivaloylanhydrid der Formel I, welcher nach Abdestillieren des überschüssigen Pivaloylchlorides bei vermindertem Druck von 2 bis 200 mbar in reiner Form erhalten wird.
